# EUROPEAN PATENT SPECIFICATION

(11) **EP 1 129 709 B1**
(45) Date of publication and mention of the grant of the patent: **29.06.2005**
(21) Application number: 01500053.2
(22) Date of filing: 01.03.2001
(51) Int. Cl.: A61K 31/19, A61K 47/40, A61K 47/48, A61K 47/20, A61K 47/18, A61K 47/12, A61K 9/14

(54) **Pharmaceutical composition based on ibuprofen and a procedure for the preparation thereof**
Pharmazeutische Zusammensetzung enthaltend Ibuprofen und ein Verfahren zur deren Herstellung
Composition pharmaceutique comprenant de l'ibuprofène et procédé pour sa préparation

(30) Priority: 03.03.2000 ES 200000522
(43) Date of publication of application: 05.09.2001
(73) Proprietor: Laboratorio de Aplicaciones Farmacodinamicas, S.A., 08025 Barcelona (ES)
(72) Inventor: Tarré Pérez, Maria Teresa, 08005 Barcelona (ES); Font Faus, Xavier, 08021 Barcelona (ES); Trullols Casas, Montserrat, 08186 Lliica d'Amunt (Barcelona) (ES)
(74) Representative: Davila Baz, Angel

(56) References cited:
- EP-A- 0 346 006
- EP-A- 0 490 193
- WO-A-95/04528
- WO-A-96/14839
- US-A- 5 597 583

## Description

This invention relates to a pharmaceutical composition based on ibuprofen with analgesic, antipyretic and anti-inflammatory activity. The invention also relates to a procedure for the preparation of said composition.

As is well known by those well versed in the art, the therapeutic use of ibuprofen, particularly administered in the form of ingestible liquid, is limited both by its low solubility and by its bitter taste, which is both disagreeable and unacceptable.

To solve this problem, different attempts have been made to hide the taste of ibuprofen and to improve its solubility. In this respect, in the state of the art there are references where procedures are described aimed at hiding the taste of ibuprofen and at increasing its solubility by means of complexing of ibuprofen with cyclodextrin (see EP 0274444, EP 0490193, WO93/20850, WO95/04528 to mention by a few references of certain relevance).

These procedures of the state of the art give rise to variable results, given that while some of them hide the taste of ibuprofen in an acceptable fashion, others do not effectively resolve the problem of the low solubility of ibuprofen and vice versa and, therefore, formulations of ibuprofen are not permitted in soluble dosage form for oral administration in the form of liquid, avoiding at the same time the bitter and unacceptable taste of ibuprofen.

In addition, the procedures of the state of the art for hiding the taste of ibuprofen and for improving its solubility are carried out by means of a number of stages that, basically, are performed in wet, with the supply of heat, drying and/or crystallisation.

As a consequence, it would be desirable to have a pharmaceutical composition based on ibuprofen with a suitable solubility of the active ingredient and which avoids the unacceptable organoleptic properties thereof.

Similarly, it would be useful to have available a procedure for the preparation of a pharmaceutical composition based on ibuprofen that avoids the drawbacks of the previous state of the art and which can be performed in a simple and economic fashion and this in order to obtain a composition in the form of a suitable powder for administration in soluble form in water, even cold water.

With this background, the present applicant carried out investigation on this topic and surprisingly, the applicant found that a combination of a soluble salt of ibuprofen and cyclodextrin, processed according to the procedure that will be described later, allowed compositions to be obtained based on ibuprofen in the form of a powder for oral administration after dissolving in water and with the taste of the active ingredient hidden.

As was mentioned earlier, ibuprofen has negative organoleptic properties. Similarly, the soluble salt of ibuprofen that is present in the composition and procedure of the invention makes these characteristics even more marked. In other words, the basic concepts of the invention are to start with an active ingredient of a salt of ibuprofen that is already soluble which, dry processed according to the invention allows the problem of the bad taste thereof to be solved. Therefore, the problem of the poor solubility of ibuprofen is solved and at the same time, the adverse organoleptic properties thereof are also eliminated.

An object of the present invention consists of improving the characteristics of solubility of the compositions based on ibuprofen present in the state of the art.

Another object of the present invention consists of improving the organoleptic characteristics of the pharmaceutical compositions based on ibuprofen present in the state of the art.

An additional object of the present invention consists of providing a pharmaceutical composition based on ibuprofen in the form of powder for oral administration in water soluble form, even in cold water, and with an agreeable taste for the patient. Yet another object of the invention consists of providing a procedure for the preparation of said pharmaceutical composition based on ibuprofen with improved organoleptic and solubility characteristics.

These and other objects of the will be evident from reading the following specification.

According to the first aspect, the invention provides a pharmaceutical composition based on ibuprofen with analgesic, antipyretic and/or anti-inflammatory activity, in the form of powder, for administration in soluble form after dissolving in water and with an acceptable taste.

According to a second aspect, the invention provides a dry procedure, without supply of heat and without drying and/or crystallisation, for the preparation of said composition.

In accordance with the first aspect of the invention, a pharmaceutical composition is provided based on ibuprofen, in the form of powder, for administration in soluble form after dissolving in water, and with an agreeable taste, characterised in that it comprises, as an active component, ibuprofen lysinate, in combination with cyclodextrin, and other pharmaceutically acceptable excipients.

It has been found that the soluble salt of ibuprofen, in other words ibuprofen lysinate, provides a greater tolerance and a faster action, which is very important taking into account the analgesic therapeutic action sought. In addition, the combination of ibuprofen lysinate with cyclodextrin has the result of hiding the disagreeable taste of said salt. This taste is suppressed in the final solution to be administered. On the other hand, the presentation in powder (single dose sachets) for dissolving in water facilitates its administration.

Preferably, ibuprofen lysinate is presented in the composition of the invention in association with β-cyclodextrin in a ratio by weight of lysinate/β-cyclodextrin of between 1:2 and 1:4.

As pharmaceutically acceptable excipients the following are used, but not exclusively: sodium saccharine, sodium cyclamate, lemon oil, sodium citrate and sucrose. As is logical, such excipients that represent a preferred embodiment of the invention can be partially or totally replaced by other excipients commonly used in the pharmaceutical sector.

Specifically, the pharmaceutical composition of the present invention comprises the following components in the following percentages by weight:
■ from 13 to 55% of mixture of ibuprofen lysinate and β-cyclodextrin;
■ from 0.10 to 0.20% of sodium saccharine;
■ from 0.15 to 0.30% of sodium cyclamate;
■ from 0.30 to 0.60% of lemon oil;
■ from 0.50 to 1.50% of sodium citrate; and
■ from 30 to 70% of sucrose.

In accordance with the second aspect of the invention, a dry procedure is provided for the preparation of the pharmaceutical composition based on ibuprofen described earlier, characterised in that it comprises the stages of:
(a) sieving the ibuprofen salt and the cyclodextrin separately;
(b) mixing said two components;
(c) submitting the resulting mixture to an ultrarapid mixing; and
(d) sieving and mixing the previous mixture along with the pharmaceutically acceptable excipients.

As has already been mentioned earlier, as an ibuprofen salt, lysinate is used and as cyclodextrin, β-cyclodextrin is used.

Preferably, the sieving of stage (a) is performed using a mesh no. 20 with a clearance of 0.80 mm.

The mixture of stage (b) is performed at a ratio by weight of ibuprofen/cyclodextrin of preferably between 1:2 and 1:4, for a time of 15 to 60 minutes.

The ultra-rapid mixing of stage (c) is carried out under shear stress and for a time of 5 to 40 minutes.

After sieving and mixing of the ibuprofen salt/cyclodextrin association with the pharmaceutically acceptable excipients, the resulting product in form of powder is packed in single-dose sachets for subsequent oral administration of the product after dissolving in water.

## Claims

1. A pharmaceutical composition based on ibuprofen, in the form of powder, for administration in soluble form in water, and with an acceptable taste, **characterised in that** it comprises, as active component, ibuprofen lysinate, in combination with β-cyclodextrin, the ratio by weight of ibuprofen lysinate/β-cyclodextrin being between 1:2 and 1:4, and other pharmaceutically acceptable excipients, selected from the group consisting of sodium saccharine, sodium cyclamate, lemon oil, sodium citrate and sucrose.

2. A composition according to claim 1, **characterised in that** it comprises the following components in the following percentages by weight:
- from 13 to 55% of mixture of ibuprofen lysinate and β-cyclodextrin;
- from 0.10 to 0.20% of sodium saccharine;
- from 0.15 to 0.30% of sodium cyclamate;
- from 0.30 to 0.60% of lemon oil;
- from 0.50 to 1.50% of sodium citrate; and from 30 to 70% of sucrose.

3. A procedure for the preparation of the pharmaceutical composition claimed in claims 1 and 2, **characterised in that** it comprises the stages of:
(a) sieving the ibuprofen salt and the β-cyclodextrin separately;
(b) mixing said two components;
(c) submitting the resulting mixture to an ultra-rapid mixing; and
(d) sieving and mixing the previous mixture along with the pharmaceutically acceptable excipients.

4. A procedure according to claim 3, **characterised in that** the sieving of stage (a) is performed using a mesh no. 20 with a clearance of 0.80 mm.

5. A procedure according to claim 3, **characterised in that** mixture of stage (b) is performed for a time of 15 to 60 minutes.

6. A procedure according to claim 3, **characterised in that** the ultra-rapid mixing of stage (c) is performed under cutting force and for a time of 5 to 40 minutes.

7. A procedure according to claims 3 to 6, **characterised in that** in stage (d) the following components are sieved and mixed in the following percentages by weight
- from 13 to 55% of mixture of ibuprofen lysinate and β-cyclodextrin;
- from 0.10 to 0.20% of sodium saccharine;
- from 0.15 to 0.30% of sodium cyclamate;
- from 0.30 to 0.60% of lemon oil;
- from 0.50 to 1.50% of sodium citrate; and
- from 30 to 70% of sucrose.

8. A procedure according to claims 3 to 7, **characterised in that** the resulting composition in powder is packaged in single-dose sachets for subsequent dissolving in water.

## Patentansprüche

1. Pharmazeutische Zusammensetzung auf der Basis von Ibuprofen, in Form von Pulver, für die Verabreichung in löslicher Form in Wasser und mit einem akzeptablen Geschmack, **dadurch gekennzeichnet, dass** sie als aktiven Bestandteil Ibuprofenlysinat in Kombination mit β-Cyclodextrin, wobei das Verhältnis von Ibuprofenlysinat/β-Cyclodextrin zwischen 1:2 und 1:4 liegt, und andere pharmazeutisch akzeptable Vehikel umfasst ausgewählt aus der Gruppe bestehend aus Natriumsaccharin, Natriumcyclamat, Zitronenöl, Natriumcitrat und Saccharose.

2. Zusammensetzung nach Anspruch 1, **dadurch gekennzeichnet, dass** sie die folgenden Bestandteile in den folgenden Gewichtsprozentsätzen umfasst:
- 13 bis 55 % der Mischung von Ibuprofenlysinat und β-Cyclodextrin;
- 0,10 bis 0,20 % Natriumsaccharin;
- 0,15 bis 0,30 % Natriumcyclamat;
- 0,30 bis 0,60 % Zitronenöl;
- 0,50 bis 1,50 % Natriumcitrat und 30 bis 70 % Saccharose.

3. Verfahren für die Zubereitung der pharmazeutischen Zusammensetzung nach den Ansprüchen 1 und 2, **dadurch gekennzeichnet, dass** sie folgende Stufen umfasst:
(a) getrenntes Sieben des Ibuprofensalzes und des β-Cyclodextrins;
(b) Mischen der beiden Bestandteile:
(c) Unterwerfen der dabei entstehenden Mischung einem ultraschnellen Mischen; und
(d) Sieben und Mischen der vorherigen Mischung zusammen mit den pharmazeutisch akzeptablen Vehikeln.

4. Verfahren nach Anspruch 3, **dadurch gekennzeichnet, dass** das Sieben der Stufe (a) mit Hilfe einer Maschengröße Nr. 20 mit einer lichten Weite von 0,80 mm durchgeführt wird.

5. Verfahren nach Anspruch 3, **dadurch gekennzeichnet, dass** das Mischen der Stufe (b) für eine Zeit von 15 bis 60 Minuten durchgeführt wird.

6. Verfahren nach Anspruch 3, **dadurch gekennzeichnet, dass** das ultraschnelle Mischen der Stufe (c) unter einer Schneidekraft und für eine Zeit von 5 bis 40 Minuten durchgeführt wird.

7. Verfahren nach den Ansprüchen 3 bis 6, **dadurch gekennzeichnet, dass** in Stufe (d) die folgenden Bestandteile gesiebt und in den folgenden Gewichsprozentsätzen gemischt werden:
- 13 bis 55 % der Mischung von Ibuprofenlysinat und β-Cyclodextrin;
- 0,10 bis 0,20 % Natriumsaccharin;
- 0,15 bis 0,30 % Natriumcyclamat;
- 0,30 bis 0,60 % Zitronenöl;
- 0,50 bis 1,50 % Natriumcitrat und
- 30 bis 70 % Saccharose.

8. Verfahren nach den Ansprüchen 3 bis 7, **dadurch gekennzeichnet, dass** die dabei entstehende Zusammensetzung in Pulverform in Eindosenbeuteln für das darauf folgende Lösen in Wasser verpackt wird.

## Revendications

1. Composition pharmaceutique basée sur l'ibuprofène, sous forme de poudre, pour l'administration sous forme soluble dans l'eau, et avec un goût acceptable, **caractérisée en ce qu'**elle comprend, en tant que composant actif, du lysinate d'ibuprofène, en combinaison avec de la β-cyclodextrine, le rapport en poids lysinate d'ibuprofène/β-cyclodextrine se trouvant entre 1:2 et 1:4, et d'autres excipients pharmaceutiquement acceptables choisis dans le groupe consistant en saccharine de sodium, cyclamate de sodium, huile de citron, citrate de sodium et sucrose.

2. Composition selon la revendication 1, **caractérisée en ce qu'**elle comprend les composants suivants dans les pourcentages en poids suivants :
- de 13 à 55% du mélange de lysinate d'ibuprofène et de β-cyclodextrine ;
- de 0,10 à 0,20% de saccharine de sodium ;
- de 0,15 à 0,30% de cyclamate de sodium ;
- de 0,30 à 0,60% d'huile de citron ;
- de 0,50 à 1,50% de citrate de sodium ; et
- de 30 à 70% de sucrose.

3. Procédé de préparation de la composition pharmaceutique revendiquée dans les revendications 1 et 2, **caractérisé en ce qu'**il comprend les étapes consistant à :
(a) tamiser le sel d'ibuprofène et la β-cyclodextrine séparément ;
(b) mélanger lesdits deux composants ;
(c) soumettre le mélange résultant à un mélange ultra-rapide ; et
(d) tamiser et mélanger le précédent mélange avec les excipients pharmaceutiquement acceptables.

4. Procédé selon la revendication 3, **caractérisé en ce que** le tamisage de l'étape (a) est réalisé en utilisant une maille n°20 avec un espacement de 0,80 mm.

5. Procédé selon la revendication 3, **caractérisé en ce que** le mélange de l'étape (b) est réalisé pendant une durée de 15 à 60 minutes.

6. Procédé selon la revendication 3, **caractérisé en ce que** le mélange ultra-rapide de l'étape (c) est réalisé sous force de cisaillement et pendant une durée de 5 à 40 minutes

7. Procédé selon une ou plusieurs des revendications 3 à 6, **caractérisé en ce que**, à l'étape (d), les composants suivants sont tamisés et mélangés dans les pourcentages en poids suivants :
- de 13 à 55% du mélange de lysinate d'ibuprofène et de β-cyclodextrine ;
- de 0,10 à 0,20% de saccharine de sodium ;
- de 0,15 à 0,30% de cyclamate de sodium ;
- de 0,30 à 0,60% d'huile de citron ;
- de 0,50 à 1,50% de citrate de sodium ; et
- de 30 à 70% de sucrose.

8. Procédé selon une ou plusieurs des revendications 3 à 7, **caractérisé en ce que** le mélange résultant en poudre est emballé dans des sachets à dose unique pour être ensuite dilué dans l'eau.
